# EUROPÄISCHE PATENTSCHRIFT

(11) **EP 1 068 900 B1**
(45) Veröffentlichungstag und Bekanntmachung des Hinweises auf die Patenterteilung: **18.06.2003**
(21) Anmeldenummer: 00115180.2
(22) Anmeldetag: 13.07.2000
(51) Int. Cl.: B01J 35/08, B01J 37/02

(54) **Aktivierte Raney-Metallkatalysatoren umfassend hohle Formen**
Activated Raney-metal catalysts made of hollow bodies
Raney-catalyseur activé à base de formes creuses

(30) Priorität: 16.07.1999 DE 19933450
(43) Veröffentlichungstag der Anmeldung: 17.01.2001
(73) Patentinhaber: Degussa AG, 40474 Düsseldorf (DE); Fraunhofer-Gesellschaft zur Förderung der angewandten Forschung e.V., 80636 München (DE)
(72) Erfinder: Ostgard, Daniel, Dr., 63801 Kleinostheim (DE); Panster, Peter, Dr., 63517 Rodenbach (DE); Rehren, Claus, Dr., 63739 Aschaffenburg (DE); Berweiler, Monika, 63477 Maintal (DE); Stephani, Günther, Dr., 01454 Grosserkmannsdorf (DE); Schneider, Lother, Dr., 01640 Coswig (DE)

(56) Entgegenhaltungen:
- EP-A- 0 300 543
- EP-A- 0 880 996
- DE-A- 2 353 631
- FR-A- 2 172 755
- US-A- 4 576 926

## Beschreibung

Vorliegende Erfindung betrifft aktivierte Raney-Metallkatalysatoren.

Aktivierte Metallkatalysatoren sind auf dem Gebiet der chemischen Technik als Raney-Katalysatoren bekannt. Sie werden, weitgehend in Pulverform, für eine große Anzahl von Hydrierungen, Dehydrierungen, Isomerisierungen und Hydratisierungen organischer Verbindungen verwendet. Diese pulverisierten Katalysatoren werden aus einer Legierung eines katalytisch aktiven Metalls, im vorliegenden auch als Katalysatormetall bezeichnet, mit einer weiteren legierungsbildenden Komponente, die in Alkalien löslich ist, hergestellt. Als Katalystormetalle werden hauptsächlich Nickel, Cobalt, Kupfer oder Eisen verwendet. Aluminium wird in der Regel als die legierungsbildende Komponente verwendet, welche in Alkalien löslich ist, jedoch können auch andere Komponenten benutzt werden, insbesondere Zink und Silicium oder Gemische aus diesen mit Aluminium.

Diese sogenannten Raney-Legierungen werden im allgemeinen nach dem Blockgießverfahren hergestellt. Bei diesem Verfahren wird ein Gemisch des Katalysatormetalls und beispielsweise Aluminium zuerst geschmolzen und in Blöcke vergossen. Typische Legierungsansätze in einem Produktionsmaßstab betragen etwa 10 bis 100 kg pro Block. Gemäß DE 21 59 736 werden Kühlzeiten von bis zu 2 Stunden erhalten. Dies entspricht einer mittleren Abkühlungsgeschwindigkeit von etwa 0,2/Sek. Demgegenüber werden bei Verfahren, wo man ein schnelles Abkühlen anwendet (beispielsweise ein Zerstäubungsverfahren) Geschwindigkeiten von 102 bis 106 K/Sek. erreicht. Die Abkühlungsgeschwindigkeit wird insbesondere durch die Teilchengröße und das Kühlmedium beeinflusst (vgl. Materialwissenschaft und -technologie, herausgegeben von R. W. Chan., P. Haasen, E. J. Kramer, Band 15, Verarbeitung von Metallen und Legierungen, 1991 VCH-Verlag Weinheim, Seiten 57 bis 110). Ein Verfahren dieser Art wird in EP 0 437 788 B1 zur Herstellung eines Raney-Legierungspulvers angewandt. Bei diesem Verfahren wird die geschmolzene Legierung bei einer Temperatur von 50 bis 500°C oberhalb ihres Schmelzpunkts zerstäubt und unter Verwendung von Wasser und/oder einem Gas abgekühlt.

Zur Herstellung eines Katalysators wird die Raney-Legierung, wenn sie nicht in der erwünschten Pulverform während der Herstellung anfiel, zuerst fein vermahlen. Sodann wird das Aluminium durch Extraktion mit Alkalien, wie z. Bsp. Natronlauge, ganz oder teilweise extrahiert. Hierdurch wird das Legierungspulver aktiviert. Nach der Extraktion des Aluminiums besitzt das Legierungspulver eine hohe spezifische Oberfläche (BET-Wert) von 20 bis 100 m²/g und ist reich an aktivem Wasserstoff. Das aktivierte Katalysatorpulver ist pyrophor und wird unter Wasser oder organischen Lösungsmitteln gelagert oder in organische Verbindungen, welche bei Raumtemperatur fest sind, eingebettet.

Pulverisierte Katalysatoren weisen den Nachteil auf, dass sie nur in einem diskontinuierlichen Verfahren verwendet werden können und nach der katalytischen Umsetzung aus dem Reaktionsmedium durch kostspieliges Absetzen und/oder Filtration abgetrennt werden müssen. Demgemäß wurde eine Vielzahl von Verfahren zur Herstellung von Formkörpern, die zu aktivierten Metallfestbettkatalysatoren nach der Extraktion des Aluminiums führen, offenbart. So sind z. Bsp. grobe teilchenförmige Raney-Legierungen, d.h., Raney-Legierungen, die lediglich grob vermahlen wurden, erhältlich, und diese können durch eine Behandlung mit Natronlauge aktiviert werden. Die Extraktion und Aktivierung verläuft sodann nur in einer Oberflächenschicht, deren Dicke durch die während der Extraktion angewandten Bedingungen eingestellt werden kann.

Ein wesentlicher Nachteil von nach diesem Verfahren des Stands der Technik hergestellten Katalysatoren sind die geringe mechanische Stabilität der aktivierten Außenschicht. Da lediglich diese Außenschicht der Katalysatoren katalytisch aktiv ist, führt ein Abrieb zur schnellen Desaktivierung, und eine neuerliche Aktivierung tiefer liegender Schichten der Legierung unter Verwendung von Natronlauge führt sodann bestenfalls zu einer partiellen Reaktivierung.

Die Patentanmeldung EP 0648 534 B1 beschreibt ausgeformte aktivierte Raney-Metallfestbettkatalysatoren und deren Herstellung. Bei diesen Katalysatoren sind die zuvor beschriebenen Nachteile vermieden, wie z. Bsp. die schlechte mechanische Stabilität, welche vom Aktivieren einer Außenschicht herrührt. Zur Herstellung dieser Katalysatoren wird ein Gemisch von Pulvern einer Katalysatorlegierung und eines Bindemittels benutzt, wobei jede der Katalysatorlegierungen mindestens ein katalytisch aktives Katalysatormetall und eine extrahierbare legierungsbildende Komponente enthält. Die reinen Katalysatormetalle oder deren Gemische, welche keine extrahierbaren Komponenten enthalten, sind als Bindemittel verwendet. Die Verwendung des Bindemittels in einer Menge von 0,5 bis 20 Gew.%, bezogen auf die Katalysatorlegierung, ist wesentlich, um nach der Aktivierung eine ausreichende mechanische Stabilität zu erreichen. Nach Ausformen der Katalysatorlegierung und des Bindemittels mit herkömmlichen Formungsmitteln und Porenbildnern werden die erhaltenen frisch hergestellten Formkörper bei Temperaturen unterhalb 850°C kalziniert. Als Ergebnis von Sintervorgängen in dem fein verteilten Bindemittel werden zwischen den einzelnen Granalien der Katalysatorlegierung feste Verbindungen erhalten. Diese Verbindungen sind im Gegensatz zu den Katalysatorlegierungen nicht oder nur in geringem Ausmaß extrahierbar, so dass eine mechanisch stabile Struktur auch nach dem Aktivieren erhalten wird. Jedoch besitzt das zugegebene Bindemittel den Nachteil, dass es im wesentlichen katalytisch inaktiv ist; infolgedessen ist die Anzahl von aktiven Zentren in der aktivierten Schicht vermindert. Zusätzlich bedeutet die absolut wesentliche Verwendung eines Bindemittels, das nur ein beschränkter Mengenbereich von Porenbildnern benutzt werden kann, ohne die Festigkeit des Formkörpers zu gefährden. Aus diesem Grund kann die Schüttdichte dieser Katalysatoren auf einen Wert von weniger als 1,9 kg pro Liter nicht vermindert werden, ohne dass ein Festigkeitsverlust auftritt. Dies führt zu einem beträchtlichen wirtschaftlichen Nachteil, wenn diese Katalysatoren in industriellen Verfahren eingesetzt werden. Insbesondere wenn kostspieligere Katalysatorlegierungen, beispielsweise Cobaltlegierungen, verwendet werden, führt die hohe Schüttdichte zu einer hohen Investition pro Reaktorbett, was jedoch durch die hohe Aktivität und Langzeitstabilität dieser Katalysatoren teilweise ausgeglichen wird. In bestimmten Fällen erfordert die hohe Schüttdichte des Katalysators eine mechanisch verstärkte Reaktorbauart.

Ein Ziel vorliegender Erfindung ist deshalb die Bereitstellung aktivierter Raney-Metallkatalysatoren aus metallischen Hohlformen, bei denen die Nachteile der zuvor genannten bekannten Festbettkatalysatoren weitgehend vermieden sind.

Das zuvor genannte Ziel und weitere Ziele vorliegender Erfindung werden erreicht, indem man hohle Formen aus den gewünschten Legierungen herstellt und sie zur Herstellung des Katalysators aktiviert. Die Hauptvorteile vorliegender Erfindung sind eine niedere Schüttdichte und eine hohe Aktivität, welche diese Materialien pro Gramm Metall zeigen.

Ein Ziel vorliegender Erfindung sind aktivierte Raney-Metallkatalysatoren, umfassend hohle Formen. Vorzugsweise sind die hohlen Formen Hohlkugeln. Diese Kugeln können einen Durchmesser von 0,5 bis 20 mm und eine Wanddicke von 0,1 bis 5 mm aufweisen.

Der Kugelmantel kann eine offene Porosität bis zu 80% zeigen. Der Kugelmantel kann aus verschiedenen Schichten bestehen.

Die Hohlkörper umfassenden Raney-Metallkatalysatoren sind aktiviert.

Ein weiteres Ziel der Erfindung ist ein Verfahren zur Herstellung der aktivierten Raney-Metallkatalysatoren, umfassend das Aufsprühen von Metallpulvern, wahlweise zusammen mit einem Bindemittel, auf Formen, welche aus einem brennbaren Material bestehen, und das Herausbrennen des Materials unter Erhalt der hohlen Form bei einer Temperatur von 450 bis 1000 °C.
Das brennbare Material kann aus Polystyrol bestehen.

Bei einer anderen Ausführungsform des erfindungsgemäßen Verfahrens zur Herstellung der aktivierten Raney-Metallkatalysatoren besteht eines der Metallpulver aus einer schnell abgekühlten Legierung. Die schnell gekühlte Legierung kann gemäß üblicherweise angewandter Verfahren gebildet werden, wie z. Bsp. durch Sprühtrocknen in verschiedenen Atmosphären, ebenso wie durch schnelles Kühlen in Flüssigkeiten, wie z. Bsp. Wasser. Die aus der Legierung und wahlweise einem Bindemittel bestehende hohle Form kann sodann mit Alkalilösungen, wie z. Bsp. Natronlauge, unter Bildung des aktivierten Katalysators aktiviert werden.

Eines der Metallpulver kann aus einer langsam abgekühlten Legierung bestehen. Die aus der Legierung und wahlweise einem Bindemittel bestehende Hohlform kann sodann mit einer Alkalilösung, wie z. Bsp. Natronlauge, unter Bildung des aktivierten Katalysators aktiviert werden.

Beim Verfahren zur Herstellung der aktivierten Raney-Metallkatalysatoren kann die Legierung aus einem oder mehreren katalytischen Metallen, wie z. Bsp. Nickel, Eisen, Kupfer, Palladium, Ruthenium und Cobalt; einer in Alkali löslichen Komponente, wie z. Bsp. Aluminium, Zink und Siliciumdioxids; und wahlweise aus einem oder mehreren Promotorelementen, wie z. Bsp. aus Cr, Fe, Ti, V, Ta, Mo, Mg, Co und/oder W bestehen.

Die erfindungsgemäßem Hohlkugeln können gemäß Andersen, Schneider und Stephani (vgl. "Neue Hochporöse Metallische Werkstoffe", Ingenieur-Werkstoffe, 4, 1998, S. 36-38) hergestellt werden. Bei diesem Verfahren wird ein Gemisch der erwünschten Legierung, eines organischen Bindemittels und wahlweise eines anorganischen Bindemittels gleichmäßig durch ein Fließbett aus Polystyrolkugeln gesprüht, wo es die Kugeln beschichtet. Die beschichteten Kugeln werden sodann bei wahlweisen Temperaturen im Bereich von 450 bis 1.000°C kalziniert, um das Polystyrol herauszubrennen, gefolgt von einer höheren Kalzinierungstemperatur, um das Metall zusammenzusintern und die Hohlform stabiler zu machen. Nach dem Kalzinieren wird der Katalysator durch Natronlauge aktiviert, um den aktivierten Grundmetallkatalysator herzustellen. Ein zusätzlicher Vorteil dieses Katalysatorsystems ist, dass man die Dicke der Wände der Hohlformen durch die Beschichtungsbedingungen und die Porosität der Wand durch die Teilchengröße und Zusammensetzung des ursprünglichen Pulvergemischs leicht steuern kann.

Für hoch aktive Katalysatoren ist die Schüttdichte des erhaltenen Festbettkatalysators sehr wichtig. Während die bekannten aktivierten Standardfestbettkatalysatoren auf Metallbasis Schüttdichten im Bereich von 2,4 bis 1,8 kg/Liter aufweisen, sind anderen Festbettanwendungen ähnliche Schüttdichten von z. Bsp. 0,3 bis 1,0 kg/Liter im hohen Maß erwünscht, um die Füllkosten eines kommerziellen Reaktors auf einem Minimum zu halten.

Das Gewichtsverhältnis von Katalysatormetall zur extrahierbaren legierungsbildenden Komponente in der Katalysatorlegierung liegt, wie es bei Raney-Legierungen herkömmlich ist, im Bereich von 20:80 bis 80:20.
Erfindungsgemäße aktivierte Raney-Metallkatalysatoren können auch mit anderen Metallen dotiert werden, um eine Wirksamkeit auf die katalytischen Eigenschaften auszuüben. Der Zweck dieser Art der Dotierung ist z. Bsp. die Verbesserung der Selektivität bei einer speziellen Umsetzung. Dotierungsmetalle werden häufig auch als Promotoren bezeichnet. Das Dotieren von Raney-Katalysatoren bzw. der Einsatz von Promotoren hierbei ist z. Bsp. im U.S.-Patent 4.153.578, in DE-AS 21 01 856, DE-OS 21 00 373 und in der DE-AS 2053799 beschrieben.

Grundsätzlich kann jede bekannte Metalllegierung, wie z. Bsp. Nickel-, Aluminium, Cobalt-Aluminium, Kupfer-Aluminium, Nickel-Chrom-, Eisen-Aluminium benutzt werden. D.h., jede der Legierungen vom Raney-Typ, bei der die Kombination von auslaugbaren Materialien, wie z. Bsp. Zink, Silicium und/oder Aluminium, zusammen mit katalytischen Materialien, wie z. Bsp. Nickel, Cobalt, Kupfer und/oder Eisen, vorliegt, kann verwendet werden.

Die Legierungen können Dotierungsmaterialien wie Chrom, Eisen, Titan, Vanadium, Tantal, enthalten; als extrahierbare Elemente können z. Bsp. Aluminium, Zink und Silicium bei vorliegender Erfindung benutzt werden. Geeignete Promotoren sind die Übergangselemente in den Gruppen IIIB bis VIIB und VIII sowie der Gruppe IB des Periodensystems der Elemente und auch die Seltene Erden-Metalle. Sie werden auch in einer Menge von bis zu 20 Gew.%, bezogen auf das Gesamtgewicht des Katalysators, angewandt. Als Promotoren werden Chrom, Mangan, Eisen, Cobalt, Vanadium, Tantal, Titan, Wolfram und/oder Molybdän sowie Metalle der Platingruppe vorzugsweise verwendet. Sie werden zweckdienlich als Legierungsbestandteile in der Katalysatorlegierung zugesetzt. Ferner können Promotoren mit einer unterschiedlichen extrahierbaren Metalllegierung in Form eines abtrennbaren Metallpulvers benutzt werden, oder die Promotoren können später auf das Katalysatormaterial angewandt werden. Eine spätere Anwendung von Promotoren kann entweder nach dem Kalzinieren oder nach dem Aktivieren erfolgen. Eine optimale Einstellung der Katalysatoreigenschaften auf das spezielle katalytische Verfahren ist auf diese Weise möglich.

Die Vorläufer des Katalysators vom Raney-Typ, die aus der Kalzinierung resultieren, sind nicht pyrophor und können ohne Schwierigkeit gehandhabt und transportiert werden. Die Aktivierung kann durch den Benutzer kurz vor der Verwendung durchgeführt werden. Eine Lagerung unter Wasser oder organischen Lösungsmitteln oder das Einbetten in organische Verbindungen ist für die Katalysatorvorläufer nicht erforderlich.

Die erfindungsgemäßen aktivierten Raney-Metallkatalysatoren können zur Hydrierung, Dehydrierung, Isomerisierung und/oder Hydratisierung organischer Verbindungen benutzt werden.

### VERGLEICHSBEISPIEL 1

Gemäß den Vorschriften in EP 0 6 48 534 A1 wurde für ein Vergleichskatalysator, der aus 1.000 g Legierungspulver aus 53% Ni und 47% Al, 100 g reinem Nickelpulver (99% Nickel, und d50=21 µm) und 25 g Ethylenbis-stearoylamid bestand, unter Zugabe von etwa 150 g Wasser ein freifließendes pelletisierbares Katalysatorgemisch hergestellt. Aus diesem Gemisch wurden Tabletten mit einem Durchmesser von 4 mm und einer Dicke von 4 mm gepresst. Die Formkörper wurden 2 Stunden bei 700°C kalziniert. Die Tabletten wurden in 20%iger Natronlauge 2 Stunden bei 80°C nach dem Kalzinieren aktiviert. Unter den Bedingungen des Anwendungsbeispiels begann dieser Katalysator Nitrobenzol bei 120°C zu hydrieren, und die Aktivität betrug 1,36 ml verbrauchten Wasserstoffs pro Gramm Katalysator pro Minute.

### BEISPIEL 1

Eine Beschichtungslösung wurde hergestellt, indem man 600 g einer schnell abgekühlten Legierung aus 50% Ni/50%Al in 800 ml einer wässerigen Lösung mit einem Gehalt an 5 Gew.% Polyvinylalkohol und 1,25 Gew.% Glycerin suspendierte. Diese Suspension wurde sodann auf 1.500 ml Kugeln aus Polystyrol (styrofoam) mit einem Bereich von 4 bis 5 mm aufgesprüht, während sie in einem nach oben gerichteten Luftstrom suspendiert waren. Nach Beschichten der Kugeln mit der zuvor erwähnten Lösung wurden die Kugeln in nach oben strömender Luft bei Temperaturen von bis zu 80°C getrocknet (es können auch höhere Temperaturen angewandt werden). Diese getrockneten, beschichteten Polystyrolkugeln hatten eine Schüttdichte von 0,45 g/ml; die Hälfte dieser Kugeln würde weiter mit einer Legierungslösung beschichtet, um die Flexibilität dieses Verfahrens zu beweisen. Die Lösung für die zweite Schicht bestand aus 700 g einer schnell abgekühlten Legierung aus 50% Ni/50% Al, die in 800 ml einer wässerigen Lösung mit einem Gehalt an 5 Gew.% Polyvinylalkohol und 1,25 Gew.% Glycerin suspendiert war. Diese Suspension wurde nun auf 750 ml der mit Ni/Al vorbeschichteten und getrockneten vorgenannten Kugeln aus Polystyrol gesprüht, während diese in einem nach oben gerichteten Luftstrom suspendiert waren. Nach dem Beschichten der Polystyrolkugeln mit der zuvor genannten Lösung wurden die Kugeln sodann in nach oben strömender Luft bei Temperaturen von bis zu 80°C getrocknet (höhere Temperaturen können auch angewandt werden). Obgleich die Lösung für die zweite Schicht der ersten ähnlich war, zeigt diese Technik klar die Fähigkeit vorliegenden Verfahrens zur Herstellung beschichteter Hohlkugeln. Die getrockneten beschichteten Kugeln wurden sodann in einem gesteuerten Stickstoff/Luftstrom bei 830°C eine Stunde erwärmt, um das Polystyrol herauszubrennen und die Legierungsteilchen zusammenzusintern. Die Hohlkugeln wurden sodann in einer 20 gew.%igen Natronlauge 1,5 Stunden bei 80°C aktiviert. Die erhaltenen aktivierten Hohlkugeln hatten Durchmesser im Bereich von 5 bis 6 mm, eine Manteldicke im Bereich von 700 bis 1.000 µ, eine Druckfestigkeit von 90 N und eine Schüttdichte von 0,62g/ml. Unter den Bedigungen des Anwendungsbeispiels 1 begann dieser Katalysator Nitrobenzol bei 110° bis 120°C zu hydrieren, und die Nitrobenzolaktivität des Katalysators war 1,54 ml verbrauchter Wasserstoff pro Gramm Katalysator pro Minute.

### BEISPIEL 2

Durch Suspendieren von 500 g einer schnell abgekühlten Legierung aus 50% Ni/50% Al und 37,5 g Nickelpulver in 750 ml einer wässerigen Lösung mit einem Gehalt an 5 Gew.% Polyvinylalkohol und 1,25 Gew.% Glycerin wurde eine Beschichtungslösung hergestellt. Diese Suspension wurde sodann auf 1.000 ml Polystyrolkugeln im Bereich von 4 bis 5 mm aufgesprüht, während diese in einem nach oben gerichteten Luftstrom suspendiert waren. Nach Beschichten der Polystyrolkugeln mit der zuvor genannten Lösung wurden die Kugeln sodann in nach oben strömender Luft bei Temperaturen von bis zu 80°C getrocknet (höhere Temperaturen können auch angewandt werden). Die getrockneten beschichteten Kugeln wurden sodann in einem gesteuerten Stickstoff/Luftstrom 1 Stunde bei 840°C erwärmt, um das Polystyrol herauszubrennen und die Nickel- und Legierungsteilchen zusammenzusintern. Die Hohlkugeln wurden sodann in einer 20 gew.%igen Natronlauge bei 80°C 1,5 Std. aktiviert. Die erhaltenen aktivierten Hohlkugeln hatten Durchmesser im Bereich von 5 bis 6 mm, eine durchschnittliche Manteldicke von 500 µ, und eine Schüttdichte von 0,34 g/ml. Unter den Bedingungen des Anwendungsbeispiels 1 begann dieser Katalysator Nitrobenzol bei 110 bis 120°C zu hydrieren, und die Nitrobenzolaktivität des Katalysators betrug 1,82 ml verbrauchter Wasserstoff pro Gramm Katalysator pro Minute.

### BEISPIEL 3

Es wurde eine Beschichtungslösung hergestellt, indem man 800 g einer Legierung aus 50% Co/50% Al in 1.000 ml wässeriger Lösung mit einem Gehalt an 5 Gew.% Polyvinylalkohol und 1,25 Gew.% Glycerin suspendierte.

Diese Suspension wurde sodann auf 2.000 ml Polystyrolkugeln im Bereich von 4 bis 5 mm aufgesprüht, während diese in einem nach oben gerichteten Luftstrom suspendiert waren. Nach dem Beschichten der Polystyrolkugeln mit der zuvor genannten Lösung wurden die Kugeln sodann in aufwärtsströmender Luft bei Temperaturen bis zu 80°C getrocknet. (Höhere Temperaturen können auch angewandt werden). Diese getrockneten, beschichteten Polystyrolkugeln hatten eine Schüttdichte von 0,35 g/ml, und die Hälfte dieser Kugeln wurde mit einer Legierungslösung weiter beschichtet. Die Lösung für die zweite Schicht bestand aus 800 g einer Legierung aus 50% Co/50% Al, die in 1.000 ml einer wässerigen Lösung mit einem Gehalt an 5 Gew.% Polyvinylalkohol und 1,25 Gew.% Glycerin suspendiert war. Diese Suspension wurde sodann auf 1.000 ml der zuvor genannten, mit Co/Al vorbeschichteten und getrockneten Polystyrolkugeln aufgesprüht, während diese in einem nach oben gerichteten Luftstrom suspendiert waren. Nach dem Beschichten der Polystyrolkugeln mit der zuvor erwähnten Lösungen wurden die Kugeln in aufwärtsströmender Luft bei Temperaturen von bis zu 80°C getrocknet (höhere Temperaturen können auch angewandt werden). Die getrockneten beschichteten Kugeln wurden sodann in einem gesteuerten Stickstoff/Luftstrom bei 700°C erwärmt, um das Polystyrol herauszubrennen und die Legierungsteilchen zusammenzusintern. Die Hohlkugeln wurden sodann in einer 20 gew.%igen Natronlauge 1,5 Std. bei 80°C aktiviert. Die erhaltenen aktivierten Hohlkugeln hatten Durchmesser im Bereich von 5 bis 6 mm, eine Manteldicke von 700 µ, eine Druckfestigkeit von 71 N und eine Schüttdichte von 0,50 g/ml. Wie aus der Entwicklung von Wasserstoffblasen visuell zu sehen war, hatte der Katalysator ein großes Reservoir aktiven Wasserstoffs.

### BEISPIEL 4

Durch Suspendieren von 800 g einer Legierung aus 50% Cu/50% Al und 104 g Kupferpulver in 1.000 ml wässeriger Lösung mit einem Gehalt an 5 Gew.% Polyvinylalkohol und 1,25 Gew.% Glycerin wurde eine Beschichtungslösung hergestellt. Diese Suspension wurde sodann auf 2.000 ml Polystyrolkugeln im Bereich von 4 bis 5 mm aufgesprüht, während diese in nach oben strömender Luft suspendiert waren. Nach Beschichten der Polystyrolkugeln mit der zuvor genannten Lösung wurden die Kugeln in aufwärts strömender Luft bei Temperaturen von bis zu 80°C getrocknet (höhere Temperaturen können auch angewandt werden). Diese getrockneten, beschichteten Polystyrolkugeln hatten eine Schüttdichte von 0,26 g/ml, und die Hälfte dieser Kugeln wurde mit einer Legierungslösung weiter beschichtet. Die Lösung für die zweite Schicht bestand aus 800 g einer Legierung aus 50% Cu/50% Al und 104 g Kupferpulver, das in 1.000 ml wässeriger Lösung mit einem Gehalt an 5 Gew.% Polyvinylalkohol und 1,25 Gew.% Glycerin suspendiert war. Diese Suspension wurde sodann auf 1.000 ml der mit Cu/Al vorbeschichteten und getrockneten, zuvor erwähnten Polystyrolkugeln aufgesprüht, während diese in einem aufwärts gerichteten Luftstrom suspendiert waren. Nach Beschichten der Polystyrolkugeln mit der zuvor genannten Lösung wurden die Kugeln in aufwärts strömender Luft bei Temperaturen von bis zu 80°C getrocknet (es können auch höhere Temperaturen angewandt werden). Die getrockneten, beschichteten Kugeln wurden sodann in einem gesteuerten Stickstoff/Luftstrom bei 550°C zum Herausbrennen des Styropors erwärmt und um das Kupfer und die Legierungsteilchen zusammenzusintern. Die Hohlkugeln wurden sodann in einer 20 gew.%igen Natronlauge bei 80°C 1,5 Stunden aktiviert. Die erhaltenen aktivierten Hohlkugeln hatten einen durchschnittlichen Durchmesser von 6 mm, eine Manteldicke im Bereich von 600 bis 700 µ und eine Schüttdichte von 0,60 g/ml. Wie aus der Entwicklung von Wasserstoffblasen visuell ersichtlich war, hatte der Katalysator ein großes Reservoir an aktivem Sauerstoff.

### BEISPIEL 5

Es wurde eine Beschichtungslösung hergestellt, indem man 800 g einer langsam abgekühlten Legierung aus 50% Ni/0,5% Fe/1,2% Cr/48,3% Al und 60 g Nickelpulver in 1.000 ml einer wässerigen Lösung mit einem Gehalt an 5 Gew.% Polyvinylalkohol und 1,25 Gew.% Glycerin suspendierte. Diese Suspension wurde sodann auf 2.000 ml Polystyrolkugeln im Bereich von 4 bis 5 mm aufgesprüht, während diese in einem nach oben gerichteten Luftstrom suspendiert waren. Nach dem Beschichten der Polystyrolkugeln mit der zuvor genannten Lösung wurden die Kugeln in aufwärts strömender Luft bei Temperaturen von bis zu 80°C getrocknet (es können auch höhere Temperaturen angewandt werden). Diese getrockneten, beschichteten Polystyrolkugeln hatten eine Schüttdichte von 0,30 g/ml, und die Hälfte dieser Kugeln wurde weiter mit einer Legierungslösung beschichtet. Die Lösung für die zweite Schicht bestand aus 800 g einer langsam abgekühlten Legierung aus 50 % Ni/0,5% Fe/1,2% Cr/48,3% Al und 60 g Nickelpulver, das in 1.000 ml einer wässerigen Lösung mit einem Gehalt an 5 Gew.% Polyvinylalkohol und 1,25 Gew.% Glycerin suspendiert war. Diese Suspension wurde sodann auf 1.000 ml der mit Ni/Fe/Cr/Al vorbeschichteten und getrockneten, zuvor erwähnten Polystyrolkugeln aufgesprüht, während diese in einem nach oben gerichteten Luftstrom suspendiert waren. Nach dem Beschichten der Polystyrolkugeln mit der zuvor genannten Lösung wurden die Kugeln in aufwärts strömender Luft bei Temperaturen von bis zu 80°C getrocknet (höhere Temperaturen können auch angewandt werden). Die getrockneten, beschichteten Kugeln wurden sodann in einem gesteuerten Stickstoff/Luftstrom bei 700°C erwärmt, um das Styropor herauszubrennen und die Nickel- und Legierungsteilchen zusammenzusintern. Die Hohlkugeln wurden sodann in einer 20gew.%igen Natronlauge 1,5 Std. bei 80°C aktiviert. Die erhaltenen aktivierten Hohlkugeln hatten einen mittleren Durchmesser von 5,9 mm, eine Manteldicke von 700 µ, eine Druckfestigkeit von 85 N und eine Schüttdichte von 0,55 g/ml. Unter den Bedingungen des Anwendungsbeispiels 1 begann dieser Katalysator Nitrobenzol bei 110°C zu hydrieren, und die Nitrobenzolaktivität des Katalysators betrug 2,4 ml verbrauchter Wasserstoff pro Gramm pro Katalysator pro Minute.

### BEISPIEL 6

Durch Suspendieren von 1.000 g einer schnell abgekühlten Legierung aus 50% Ni/50% Al und 75 g Nickelpulver in 1.000 ml einer wässerigen Lösung mit einem Gehalt an 5 Gew.% Polyvinylalkohol und 1,25 Gew.% Glycerin wurde eine Beschichtungslösung hergestellt. Diese Suspension wurde sodann auf 2.000 ml Polystyrolkugeln im Bereich von 2 bis 3 mm aufgesprüht, während diese in einem aufwärts gerichteten Luftstrom suspendiert waren. Nach Beschichten der Polystyrolkugeln mit der zuvor genannten Lösung wurden die Kugeln in aufwärts strömender Luft bei Temperaturen von bis zu 80°C getrocknet (höhere Temperaturen können auch angewandt werden). Diese getrockneten, beschichteten Polystyrolkugeln hatten eine Schüttdichte von 0,33 g/ml, und die Hälfte dieser Kugeln wurden mit einer Legierungslösung weiter beschichtet. Die Lösung für die zweite Schicht bestand aus 1.000 g einer schnell abgekühlten Legierung aus 50% Ni/50% Al und 75 g Nickelpulver, das in 1.000 ml einer wässerigen Lösung mit einem Gehalt an 5 Gew.% Polyvinylalkohol und 1,25 Gew.% Glycerin suspendiert war. Diese Suspension wurde sodann auf 1.000 ml der mit Ni/Al vorbeschichteten und getrockneten, zuvor erwähnten Polystyrolkugeln aufgesprüht, während diese in einem nach oben gerichteten Luftstrom suspendiert waren. Nach dem Beschichten der Polystyrolkugeln mit der zuvor erwähnten Lösung wurden die Kugeln in aufwärts strömender Luft bei Temperaturen von bis zu 80°C getrocknet (höhere Temperaturen können auch angewandt werden). Diese getrockneten, doppelt beschichteten Polystyrolkugeln hatten eine Schüttdichte von 0,75 g/ml, und die Hälfte dieser Kugeln wurde nochmals mit einer dritten Zugabe der Legierungslösung beschichtet. Die Lösung für die dritte Schicht bestand aus 1.000 g einer schnell abgekühlten Legierung aus 50% Ni/50% Al und 75 g Nickelpulver, die in 1.000 ml wässeriger Lösung mit einem Gehalt an 5 Gew.% Polyvinylalkohol und 1,25 Gew.% Glycerin suspendiert waren. Diese Suspension wurde sodann auf 500 ml der mit Ni/Al doppelt vorbeschichteten und getrockneten, zuvor erwähnten Polystyrolkugeln aufgesprüht. Nach dem Beschichten der Polystyrolkugeln mit der zuvor genannten Lösung wurden die Kugeln in aufwärts strömender Luft bei Temperaturen von bis zu 80°C getrocknet (höhere Temperaturen können auch angewandt werden). Die getrockneten, dreifach beschichteten Kugeln wurden sodann in einem regulierten Stickstoff/Luftstrom bei 700°C erwärmt, um das Polystyrol herauszubrennen und die Nickel- und Legierungsteilchen miteinander zu versintern. Die Hohlkugeln wurden sodann in 20 gew.%iger Natronlauge 1,5 Std. bei 80°C aktiviert. Die erhaltenen aktivierten Hohlkugeln hatten einen mittleren Durchmesser von 4,5 mm, eine Manteldicke von 600 bis 700 µ und eine Schüttdichte von 0,85 g/ml. Unter den Bedingungen des Anwendungsbeispiels 1 begann dieser Katalysator Nitrobenzol bei 78°C zu hydrieren, und die Nitrobenzolaktivität des Katalysators war 3,46 ml verbrauchter Wasserstoff pro Gramm Katalysator pro Minute.

### ANWENDUNGSBEISPIEL 1

Die katalytische Aktivität des Katalysators von den Vergleichsbeispielen 1 und von den Beispielen 1 bis 5 während der Hydrierung vom Nitrobenzol wurden verglichen. Zu diesem Zweck wurden 100 g Nitrobenzol und 100 g Ethanol in einen Rührautoklaven mit einem Fassungsvermögen von 0,5 Liter gefüllt, der mit einem Gasrührer ausgestattet war. 10 g des dem Test unterworfenen Katalysators wurden jeweils in dem Rührautoklaven unter Verwendung eines Katalysatorkorbs suspendiert, so dass das Katalysatormaterial durch das Gemisch aus Reaktionsteilnehmer und Lösungsmittel sorgfältig gewaschen wurde, und Wasserstoff wurde eingeleitet. Die Hydrierung wurde bei einem Wasserstoffdruck von 40 bar und einer Temperatur von 150°C durchgeführt. Die Temperatur bei Beginn, und die Rate des Wasserstoffverbrauchs wurden ermittelt. Die Ergebnisse sind in Tabelle 1 wiedergegeben. Zur Überprüfung wurden nach 1,2,3,4 und 5 Stunden Proben entnommen und durch Gaschromatographie analysiert.

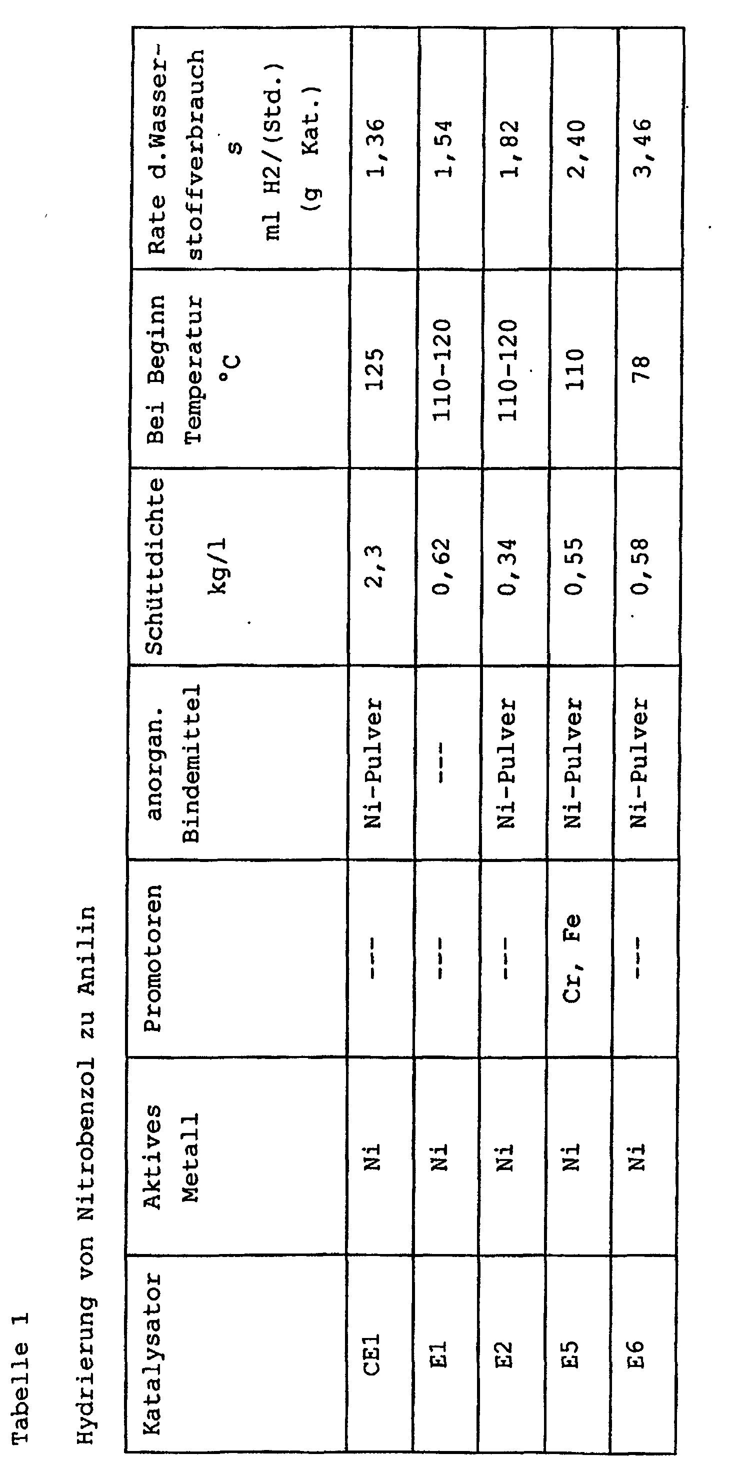

## Patentansprüche

1. Aktivierte Raney-Metallkatalysatoren, umfassend hohle Formen.

2. Aktivierte Raney-Metallkatalysatoren gemäß Anspruch 1, umfassend Hohlkugeln.

3. Aktivierte Raney-Metallkatalysatoren gemäß Anspruch 2, umfassend einen Durchmesser von 0,5 bis 20 mm und eine Wanddicke von 0,1 bis 5 mm.

4. Aktivierte Raney-Metallkatalysatoren gemäß Anspruch 2, umfassend einen Mantel, der eine offene Porosität von maximal etwa 80% aufweist.

5. Aktivierte Raney-Metallkatalysatoren gemäß Anspruch 2, umfassend einen Mantel, der aus verschiedenen Schichten besteht.

6. Verfahren zur Herstellung der aktivierten Raney-Metallkatalysatoren gemäß Anspruch 1, umfassend das Aufsprühen von Metallpulvern, wahlweise zusammen mit einem Bindemittel, auf Formen, die aus einem brennbaren Material besteht, und das Herausbrennen des Materials unter Erhalt der hohlen Form bei einer Temperatur von 450 bis 1000 °C.

7. Verfahren zur Herstellung der aktivierten Raney-Metallkatalysatoren gemäß Anspruch 4 und 6, wobei eines der Metallpulver aus einer schnell abgekühlten Legierung besteht.

8. Verfahren zur Herstellung der aktivierten Raney-Metallkatalysatoren gemäß Ansprüchen 1 und 6, wobei eines der Metallpulver aus einer langsam abgekühlten Legierung besteht.

9. Verfahren zur Herstellung der aktivierten Raney-Metallkatalysatoren gemäß Anspruch 6, wobei das brennbare Material aus Polystyrol besteht.

10. Verfahren zur Herstellung der aktivierten Raney-Metallkatalysatoren gemäß Anspruch 7 und 8, wobei die Legierung aus einem oder mehreren katalytischen Metallen, einer alkalilöslichen Komponente und wahlweise aus einem oder mehreren Promotorelementen besteht.

11. Verwendung der Metallkatalysatoren gemäß Anspruch 1 zur Hydrierung, Dehydrierung, Isomerisierung und/oder Hydratisierung von organischen Verbindungen.

## Claims

1. Activated Raney-metal catalysts, comprising hollow bodies.

2. Activated Raney-metal catalysts according to claim 1, comprising hollow spheres.

3. Activated Raney-metal catalysts according to claim 2, comprising a diameter of 0.5 to 20 mm and a wall thickness of 0.1 to 5 mm.

4. Activated Raney-metal catalysts according to claim 2, comprising a shell that has a maximum open porosity of about 80%.

5. Activated Raney-metal catalysts according to claim 2, comprising a shell that consists of different layers.

6. Process for the production of the activated Raney-metal catalysts according to claim 1, comprising the spraying of metal powders, optionally together with a binder, on to moulds that consist of a flammable material, and the burning out of the material to obtain the hollow body at a temperature of 450 to 1000°C.

7. Process for the production of the activated Raney-metal catalysts according to claim 4 and 6, wherein the metal powder consists of a rapidly-cooled alloy.

8. Process for the production of the activated Raney-metal catalysts according to claims 1 and 6, wherein one of the metal powders consists of a slowly-cooled alloy.

9. Process of the production of the activated Raney-metal catalysts according to claim 6, wherein the flammable material consists of polystyrene.

10. Process for the production of the activated Raney-metal catalysts according to claim 7 and 8, wherein the alloy consists of one or more catalytic metals, an alkali-soluble component and optionally one or more promoter elements.

11. Use of the metal catalysts according to claim 1 for the hydrogenation, dehydrogenation, isomerisation and/or hydration of organic compounds.

## Revendications

1. Catalyseurs en métal de Raney activés, comprenant des formes creuses.

2. Catalyseurs en métal de Raney activé selon la revendication 1 comprenant des billes creuses.

3. Catalyseurs en métal de Raney activé selon la revendication 2 comprenant un diamètre de 0,5 à 20 mm et une épaisseur de paroi de 0,1 à 5 mm.

4. Catalyseurs en métal de Raney activés selon la revendication 2 comprenant une enveloppe qui présente une porosité ouverte d'environ 80 % au maximum.

5. Catalyseurs en métal de Raney activés selon la revendication 2, comprenant une enveloppe qui se compose de différentes couches.

6. Procédé de production des catalyseurs en métal de Raney activés selon la revendication 1, comprenant la pulvérisation de poudres métalliques, au choix avec un liant, sur des formes qui se composent d'un matériau combustible, et la combustion du matériau avec maintien de la forme creuse à une température de 450 à 1000°C.

7. Procédé de production des catalyseurs de métal de Raney activés selon les revendications 4 et 6, dans lequel la poudre métallique se compose d'un alliage rapidement refroidi.

8. Procédé de production des catalyseurs en métal de Raney activés selon les revendications 1 et 6, dans lequel l'une des poudres métalliques se compose d'un alliage lentement refroidi.

9. Procédé de production des catalyseurs en métal de Raney activé selon la revendication 6, dans lequel le matériau combustible se compose de polystyrène.

10. Procédé de production des catalyseurs en métal de Raney activés, selon la revendication 7 et 8, dans lequel l'alliage se compose d'un ou plusieurs métaux catalytiques, d'un composant soluble dans les bases et au choix d'un ou plusieurs éléments promoteurs.

11. Utilisation des catalyseurs métalliques selon la revendication 1 pour l'hydrogénation, la déshydrogénation, l'isomérisation et/ou l'hydratation de composés organiques.
